Europäisches Patentamt

European Patent Office

Office européen des brevets

(19)

(11) Publication number : **0 383 405 B1**

(12) **EUROPEAN PATENT SPECIFICATION**

(45) Date of publication of patent specification :
30.06.93 Bulletin 93/26

(51) Int. Cl.⁵ : **C12P 7/62, C12P 7/64**

(21) Application number : **90200337.5**

(22) Date of filing : **14.02.90**

(54) **Ester preparation.**

The file contains technical information
submitted after the application was filed and
not included in this specification

(30) Priority : **17.02.89 EP 89200377**

(43) Date of publication of application :
**22.08.90 Bulletin 90/34**

(45) Publication of the grant of the patent :
**30.06.93 Bulletin 93/26**

(84) Designated Contracting States :
**AT BE CH DE DK ES FR GB GR IT LI NL SE**

(56) References cited :
**EP-A- 0 241 094**
**EP-A- 0 274 798**
**WO-A-86/05186**
**WO-A-89/01032**

(73) Proprietor : **UNICHEMA CHEMIE BV**
**Buurtje 1**
**NL-2802 BE Gouda (NL)**

(72) Inventor : **Hills, Geoffrey Alan**
**Kornwestheimerstr. 8a**
**W-7000 Stuttgart 40 (DE)**
Inventor : **Macrae, Alasdair Robin**
**Ivy Cottage, Newton Blossomville**
**Bedford MK43 8AN (GB)**
Inventor : **Poulina, Ramires Rolando**
**Anna van Hensbeeksingel 162**
**NL-2803 LK Gouda (NL)**

(74) Representative : **Matthews, Heather Clare et al**
**Keith W Nash & Co Pearl Assurance House**
**90-92 Regent Street**
**Cambridge CB2 1DP (GB)**

## Description

The invention relates to a process for the preparation of an ester by reacting a C7 - C36 mono- or dicarboxylic acid and a C2 - C8 monoalcohol in the presence of a lipase catalyst.

Processes for the preparation of esters using similar starting materials are known in the art. Most of them employ chemical esterification catalysts such as organic sulfonic acids e.g. p.toluenesulfonic acid, metal alkylates e.g. tetrabutyltin etc. The esters so obtained therefore also contain catalyst residues and by-products as well as side-products such as ethers which are difficult to remove. Less contaminated esters, which are desirable especially for pharmaceutical and cosmetic applications may be obtained by preparing the esters by enzymatic processes but generally these processes suffer from one or more of the following disadvantages: low yield of ester, high consumption of enzyme, handling difficulties etc.

The following citations disclose enzymatic esterification processes:

JAOCS, Vol 65, no 6 June 1988 pp 927-31 which discusses the use of immobilized lipase for the commercial synthesis of esters and states that the effect of water in these reactions is important because on the one hand the presence of water is required to avoid structural changes in the enzyme whereas on the other hand the absence of water is critical if a quantitative yield is required, then not only must the initial water level be low, but the formed water somehow must be removed from the reaction. It is therefore obviously difficult to compromise satisfactorily and to obtain high yields at reasonable enzyme levels. It is also stated that a relatively easy method to remove the "formed water," particularly when dealing with higher boiling reactants, is to simply run the reaction under reduced pressure. As an illustration the reaction of oleic acid with oleyl alcohol is shown to level off at 85% yield when no formed water is removed, but when water is continuously pulled off the esterification is driven to completion.

WO 88/02775 (Novo Industri A/S) also relates to lipase and to reactions with this enzyme and states on p. 14 that for ester synthesis processes from short-chain alcohols (primary or secondary) it is preferred to use <u>Canidida antarctica</u> lipase containing lipas B. For ester synthesis from long-chain, non-volatile alcohols, it is preferred to use a <u>C. antarctica</u> preparation containing lipase A and to apply vacuum for water removal.

Both citations consequently advocate the use water removal under reduced pressure when dealing with long-chain, non-volatile (ie higher boiling) reactants. The publications evidently point away from the use of water removal under reduced pressure when dealing with short-chain, volatile (ie low boiling) reactant(s).

EP-A-0274798 discloses a process for preparing an ester from an alcohol and fatty acid by enzyme-catalysed conversion in which a fatty acid is contacted with lipase and the alcohol is added at such a rate that the alcohol concentration in the reaction mixture is kept below a mole ratio of 0.5 moles of alcohol per mole of fatty acid/acyl group. Water of reaction is removed from the reaction vessel and can be removed by distillation, although there is no disclosure of its removal by azeotropic distillation. Yields of product in the examples given are typically around 70%.

W0-A-89/01032 discloses an immobilised lipase and its use in ester synthesis. Also described in this document is a process of ester synthesis in which water is removed during the reaction, eg by vacuum distillation or by absorbtion on molecular sieves. There is, however, no disclosure of use of azeotropic distillation or of ester synthesis using a C2-C8 monoalcohol.

W0-A-86/05186 discloses a process for esterification of an acetalized or ketalized polyol with a carboxylic acid. Azeotropic distillation is disclosed as one method of removing water of reaction. Typical yields are in the range 10-15%. However, there is no disclosure of use of a C2-C8 monoalcohol or of use of such a monoalcohol in the azeotropic distillation process.

According to the present invention the esterification of the C7-C36 mono- or dicarboxylic acid and C2 - C8 monoalcohol is conducted in such a way that water of reaction is removed, usually continuously, by azeotropic distillation of a mixture of water and the volatile C2 - C8 monoalcohol. This is not azeotropic distillation as is often practiced with the addition of an aromatic solvent such as benzene or toluene.

Azeotropic distillation is here understood to be distillation of a mixture of monoalcohol and water having a minimum in the boiling point curve so that the distillate will tend towards the composition of monoalcohol-water azeotropic mixture. Preferably the rate at which the alcohol is distilled off substantially equals the rate at which more alcohol is fed into the esterification reactor. The feeding of the alcohol can be continuously or stepwise.

Suitable carboxylic acids in the practice of this invention are in particular straight-chain monocarboxylic acids, such as oenanthic acid, caprylic acid, pelargonic acid, capric acid, lauric acid, myristic acid, palmitic acid, stearic acid, arachidic acid, behenic acid, lignoceric acid, cerotic acid and their mono- and poly-unsaturated counterparts. Dicarboxylic acids can also be used in the practice of the invention including dicarboxylic acids such as azelaic acid, sebacic acid, brassylic acid, dimer acid etc. can be used.

Suitable mono-alcohols are those containing 2 to 8 carbon atoms and the alcohols can be primary or secondary. Suitable alcohols are e.g. ethanol, propane-1-ol, propane-2-ol, butane-1-ol, butane-2-ol, isobutanol, hex-

EP 0 383 405 B1

ane-1-ol, 2-ethyl hexane-1-ol, octane-1-ol etc.

The process of the invention is particularly suitable for the preparation of esters of C10 - C18 monocarboxylic acids and C3 - C4 alcohols, especially secondary alcohols.

Carrying out the esterification under controlled and mild conditions is important because some of the reactants tend to form undesirable by-products especially at higher temperatures. Gradual addition of alcohol to the reaction as the alcohol-water mixture is distilled off is preferred.

Suitable lipases for the practice of this invention are those obtainable from well-known microorganisms such as Aspergillus niger, Rhizopus species, Penicillum - cyclopium, Candida species, Pseudomonas species, Mucor Miehei and Humicola species and are readily available from enzyme manufacturers. For good results it is preferred that the lipase is attached to a suitable water-insoluble inorganic or organic carrier material such as a silica, diatomaceous earth, carbon black or organic polymer such as ion exchange resins, acrylate resins, porous polypropylene, porous polystyrene etc.

The lipase can be attached to the carrier by adsorption, it can be immobilized in a matrix of carrier material or attached by a covalent bond.

Preferably the removal of water of reaction is carried out by azeotropic distillation under reduced pressure. Alcohol drier than the alcohol-water mixture distilled off is added (continuously) as the esterification progresses. More in particular the reduced pressure is in the range of 5.000 - 50.000 Pa and usually at a temperature below 80°C. More preferably esterification and azeotropic distillation are conducted in a single vessel system at a temperature below 80°C.

Especially in the case of the less volatile alcohols it is useful to carry out esterification and azeotropic distillation in separate vessels, where the temperature in the distillation vessel can be somewhat higher than in the esterification vessel.

This also opens the possibility of distilling the mixture of C2 -C8 alcohol and water under non-reduced or higher pressures. When separate vessels are used it is preferred to carry out the esterification in a packed column filled with lipase immobilized on a carrier material. Also it is possible to carry out the esterification with lipase on carrier in a fluidized bed.

In a preferred embodiment of the invention C2 - C8 monoalcohol - water distillate is distilled off and then at least part of it is returned to the esterification reactor after reduction of the water percentage. Possible procedures for reduction of the water percentage in the distillate are : phase separation, water absorbing agents (molecular sieves, silica gel), distillation (optionally with a third substance), addition of drier alcohol etc.

In order to obtain an almost theoretical conversion into ester it is recommended to progressively reduce the water percentage in the distillate over the course of reaction before returning the alcohol to the esterification reactor.

Also it recommended to dose the alcohol in such a way that in the esterification reactor there is a stoichiometric excess of total acyl groups (R-CO-) over free alcohol. Addition of alcohol is stopped when the acid value of the esterification mixture has reached an acid value below 5. When the desired end acid value has been reached the contact between the ester product and the lipase is stopped and the ester product is filtered and an ester is obtained which shows an excellent colour and no odour. Optionally this ester can be further refined by neutralization of unesterified acid, stripping with steam etc.

Example 1

Isopropylmyristate was prepared by charging myristic acid (98%; 50 kg) and propane-2-ol (13 kg) into a 100 litre stirred tank reactor equipped with heating coils, condensor and vacuum facility. The reactants were heated with stirring to 60°C and 1 kg lipase enzyme (code SP382, ex NOVO Industri, lipase catalyst from Candida species immobilised on acrylate beads) was added. The reaction was continued at 60°C and 20.000 Pa. pressure by dosing further propane-2-ol at 5 kg/hr to replace the distilling propane-2-ol/water azeotrope, until the acid value of the product was 0.5. Excess propane-2-ol was then removed under reduced pressure and the product isopropyl myristate separated from the enzyme by filtration. The resulting product showed excellent colour (10 APHA) and had no odour. Product yield was better than 99%.

Example 2

A reaction mixture was made up by stirring together decanoic acid, 187g, and propane-2-ol (65g), in a reservoir at a temperature of 80°C. Liquid from the reservoir was pumped continuously through a 5g bed of Novo SP382 lipase catalyst (from Candida species immobilised on acrylate resin beads), maintained at a temperature of 60°C, at a flow rate of 150ml/hr, and then returned to the reservoir. At the same time, to remove the water produced by the esterification reaction in the packed bed column reactor, propane-2-ol was continuously

3

pumped and mixed into the reservoir at 35ml/hr and propane-2-ol/water distilled off at the same rate under a vacuum of 40.000-50.000 Pa The progress of the reaction is shown in Table 1.

After 22 hours 99.2% of the decanoic acid was esterified and the reaction was stopped. The mixture in the reservoir was collected and excess propane-2-ol removed from it under reduced pressure to give isopropyldecanoate (acid value 2.6) in 96% yield (a few percent of ester was left in the catalyst bed). The ester was colourless and odourless.

| Table 1 | Results |
|---|---|
| Reaction time (hrs) | Amount of decanoic acid esterified (%) |
| 1 | 16.7 |
| 2 | 34.1 |
| 3 | 45.1 |
| 4 | 57.5 |
| 5 | 66.0 |
| 6 | 72.9 |
| 22 | 99.2 |

Without removal of the water by azeotropic distillation a yield of only 70% is isopropyl ester was achieved.

Example 3

250g of "isostearic acid" (Prisorine (Registered Trade Mark) 3505, ex Unichema Chemie BV) and 52.8g of propan-2-ol were heated to 60°C in a stirred vessel. 6g of immobilised Candida antartica lipase catalyst (code SP382, ex Novo Industri) was added. The pressue was reduced to 20.000 Pa and propan-2-ol azeotrope distilled over.

The reaction proceeded for 20 hours, then the catalyst was removed by filtration and excess propan-2-ol was removed by rotary evaporation. Isopropyl isostearate (acid value of 6.8) was produced, at an overall yield of 97.6%.

Example 4

Di-2-Ethyl-1-hexyl azelate was produced by reacting 50g of axelaic acid with 69.2g of 2-ethyl-1-hexanol using the method described in example 3 with the following modifications. Because of the acid's poor solubility in the alcohol, the reaction temperatur was raised to 70°C and initially only 22g of azelaic acid was dissolved in 69.2g of 2-ethy-1-hexanol. 2g of immobilised Candida antartica lipase catalyst (code SP382, ex Novo Industri) was added. The rest of the azelaic acid was fed to the reaction vessel in three batches of 10g, 10g and 8g as the acid value dropped below 120. As in example 3, alcohol was fed to match the distillation rate of the 2-ethyl-1-hexanol / water azeotrope.

After 24 hours, di-2-ethyl-1-hexyl azelate (acid value of 0.3) was obtained, at a yield of 97.1%.

Example 5

Ethyl oleate was produced by reacting 283g of oleic acid (Priolene (Registered Trade Mark) 6901 ex Unichema Chemie BV) with 46g of ethanol in the presence of 5.65g of immobilised Candida antartica lipase catalyst (code SP382, ex Novo Industri) using the method described in example 3 with the following modifications. Ethanol was fed at 164g per hour to match the distillation rate of the ethanol / water azeotrope at a pressure of 81.000 Pa. After 22 hours, ethyl oleate (acid value of 1.07) was obtained, at a yield of greater than 98%.

## Example 6

N-butyl alcohol (106g) and linoleic acid (400g of Prifac (Registered Trade Mark) 7961 ex Unichema Chemie BV, 71% pure linoleic acid) were reacted together, using 8g of immobilised Candida antartica lipase catalyst (SP382, ex Novo Industri) by the method given in example 3, with the following modifications.

The n-butyl alcohol / water azeotrope was distilled off at 70,000 to 80,000 Pa. On condensing it separated into two phases. The alcohol-rich phase was returned to the reaction vessel using Dean & Stark apparatus. Fresh n-butyl alcohol was fed to the vessel during the course of the reaction to replace that lost in the water-rich phase. After 48 hours, butyl linoleate with an acid value of 5.1 was produced at an overall yield of 98.6%.

## Example 7

Butyl linoleate was prepared, as in example 6, using 200g of linoleic acid (Prifac 7961, ex Unichema Chemie BV, 53g of n-butyl alcohol and 4g of immobilised Rhizopus niveus lipase catalyst. 5g of water was added to start the reaction, which proceeded for 70 hours whilst distilling off the azeotrope to give butyl linoleate (acid value 5.5) with a yield greater than 95%.

## Example 8

78g of isobutyl alcohol was reacted with 300g of stearic acid (Prifac 2980, ex Unichema, 90% stearic acid) using 7g of immobilised Candida antartica lipase catalyst (code SP382, ex Novo Industri). The method of example 6 was used except that the azeotrope of isobutyl alcohol and water distilled over at 50.000 Pa.
After 16 hours, isobutyl stearate (acid value 0.8) was produced with a yield of 98%.

## Example 9

Hexyl laurate was produced using the method of example 6. 100g of lauric acid was reacted with 51g of hexanol using 3g of immobilised Candida antartica lipase catalyst (code SP382, ex Novo Industri). The water / hexanol azeotrope distilled over at 30.000 Pa, forming two phases. The alcohol-rich phase was recycled using a Dean & Stark separation apparatus.
After 28 hours hexyl laurate with an acid value of 1.56 was obtained at a yield of 99%.

## Example 10

100g of lauric acid and 51g of hexanol were reacted as in example 9 but using 3g of immobilised Humicola lipase catalyst (code SP378, ex Novo Industri). 3g of water was added to the reactants to start the conversion and the pressure held at 70.000 Pa to distil off the azeotrope.
After 24 hours hexyl laurate (acid value 2.7) was obtained, with a yield of 98%.

## Example 11

Ethyl esters were produced by reacting 100g of fractionated fish oil fatty acids containing 30.24% eicosapentaenoic acid and 26.67% docosahexaenoic acid (EPA-Chol 750 marine lipid concentrate, ex E.P.A. LIMITED) with 15g of ethanol in the presence of 2g of immobilised Candida antartica lipase catalyst (code SP382, ex Novo Industri) at 50.000 Pa by the method given in example 3. After 24 hours, fish oil esters (acid value 2.8) were produced at a yield of 98%.

## Example 12

300g of ricinoleic acid and 61g of propan-1-ol were reacted together in the presence of 6g of immobilised Mucor miehei (code SP392 lipase catalyst ex Novo Industri) under a reduced pressure of 40.000 - 60.000 Pa by the method of example 3. After 30 hours, propyl ricinoleate (acid value 2.5) was produced at a yield of 97.5%.

## Example 13

2.5kg of palmitic acid (Prifrac (registered Trade Mark) 2960 ex Unichema Chemie BV) and 0.58kg of propan-2-ol were heated to 60°C in a stirred vessel equipped with a condenser and vacuum facility. 60g of immobilised Candida antartica lipase catalyst (SP382, ex Novo Industri) was added. The reaction was continued at 60°C and 26,000 Pa pressure by dosing further propan-2-ol at an average rate of 58 ghr $^{-1}$ to replace distilling propan-2-ol/water azeotrope. After 14 hours the reaction was stopped, and the catalyst removed by filtration. Excess propan-2-ol was removed from the product by evaporation under reduced pressure to give isopropylpalmitate (Acid value 0.8) in an overall of 99%.

## Claims

1. A process for preparing an ester by reacting a C7 - C36 mono- or dicarboxylic acid and a C2 - C8 monoalcohol in the presence of a lipase catalyst characterized in that water of reaction is removed by azeotropic distillation of a mixture of C2 - C8 monoalcohol and water.

2. A process according to the preceding claim characterized in that the azeotropic distillation of alcohol and the addition of alcohol are carried out at substantially the same rates .

3. A process according to claim 1 or 2 characterized in that the azeotropic distillation is carried out under reduced pressure.

4. A process according to claim 1, 2 or 3 characterized in that the esterification and azeotropic distillation are conducted in a single vessel system at a temperature below 80°C.

5. A process according to any of the preceding claims characterized in that esterification and azeotropic distillation are carried out in separate vessels.

6. A process according to claim 5 characterized in that the esterification is carried out with lipase catalyst in packed column reactor.

7. A process according to claim 5 characterized in that the esterification is carried out with lipase catalyst in a fluidized bed.

8. A process according to any of the preceding claims characterized in that at least part of the C2 - C8 monoalcohol/ water distillate is returned to the reactor after removal of at least part of the water contained in the distillate.

9. A process according to any of the preceding claims characterized in that in the esterification reactor there is a stoichiometric excess of total acyl groups over free alcohol.

10. A process according to any of the preceding claims characterized in that the lipase catalyst is present in the form of lipase enzyme immobilized on a carrier material.

## Patentansprüche

1. Verfahren zur Herstellung eines Esters durch Umsetzen einer $C_7$-$C_{36}$-Mono- oder -Dicarbonsäure und eines $C_2$-$C_8$-Monoalkohols in Gegenwart eines Lipasekatalysators, dadurch gekennzeichnet, daß das Reaktionswasser durch azeotrope Destillation einer Mischung aus $C_2$-$C_8$-Monoalkohol und Wasser entfernt wird.

2. Verfahren nach dem vorhergehenden Anspruch, dadurch gekennzeichnet, daß die azeotrope Destillation des Alkohols und die Zugabe des Alkohols mit praktisch den gleichen Geschwindigkeiten durchgeführt werden.

3. Verfahren nach Anspruch 1 oder 2, dadurch gekennzeichnet, daß die azeotrope Destillation unter vermindertem Druck durchgeführt wird.

4. Verfahren nach Anspruch 1, 2 oder 3, dadurch gekennzeichnet, daß die Veresterung und die azeotrope Destillation in einem Einzelgefäßsystem bei einer Temperatur unter 80°C durchgeführt werden.

5. Verfahren nach irgendeinem der vorhergehenden Ansprüche, dadurch gekennzeichnet, daß die Veresterung und die azeotrope Destillation in getrennten Gefäßen durchgeführt werden.

6.  Verfahren nach Anspruch 5, dadurch gekennzeichnet, daß die Veresterung mit Lipasekatalystor in einem Füllkörperkolonnenreaktor durchgeführt wird.

7.  Verfahren nach Anspruch 5, dadurch gekennzeichnet, daß die Veresterung mit Lipasekatalysator in einem Wirbelbett durchgeführt wird.

8.  Verfahren nach irgendeinem der vorhergehenden Ansprüche, dadurch gekennzeichnet, daß mindestens ein Teil des $C_2$-$C_8$-Monoalkohol/Wasser-Destillates nach Entfernung mindestens eines Teils des im Destillat enthaltenen Wassers zum Reaktor zurückgeführt wird.

9.  Verfahren nach irgendeinem der vorhergehenden Ansprüche, dadurch gekennzeichnet, daß im Veresterungsreaktor ein stöchiometrischer Überschuß gesamter Acylgruppen über den freien Alkohol vorliegt.

10. Verfahren nach irgendeinem der vorhergehenden Ansprüche, dadurch gekennzeichnet, daß der Lipasekatalysator in Form eines auf einem Trägermaterial immobilisierten Lipaseenzyms vorliegt.


**Revendications**

1.  Procédé de préparation d'un ester par réaction d'un acide mono- ou di-carboxylique en $C_{7-36}$ et d'un monoalcool en $C_{2-8}$ en présence d'un catalyseur de lipase, caractérisé en ce qu'on soutire l'eau de réaction par distillation azéotropique d'un mélange de monoalcool en $C_{2-8}$ et d'eau.

2.  Procédé selon la revendication 1, caractérisé en ce qu'on effectue la distillation azéotropique de l'alcool et l'addition de l'alcool sensiblement aux mêmes débits.

3.  Procédé selon la revendication 1 ou 2, caractérisé en ce qu'on effectue la distillation azéotropique sous pression réduite.

4.  Procédé selon la revendication 1, 2 ou 3, caractérisé en ce qu'on exécute l'estérification et la distillation azéotropique dans un système à récipient unique à une température inférieure à 80°C.

5.  Procédé selon l'une quelconque des revendications précédentes, caractérisé en ce qu'on effectue l'estérification et la distillation azéotropique dans des récipients séparés.

6.  Procédé selon la revendication 5, caractérisé en ce qu'on effectue l'estérification avec un catalyseur de lipase dans un réacteur à colonne garnie.

7.  Procédé selon la revendication 5, caractérisé en ce qu'on effectue l'estérification avec un catalyseur de lipase en lit fluidisé.

8.  Procédé selon l'une quelconque des revendications précédentes, caractérisé en ce qu'on recycle au réacteur au moins une partie du distillat de monoalcool en $C_{2-8}$ et d'eau après soutirage d'une partie au moins de l'eau contenue dans le distillat.

9.  Procédé selon l'une quelconque des revendications précédentes, caractérisé en ce que dans le réacteur d'estérification, il existe un excès stoechiométrique des groupes acyliques totaux par rapport à l'alcool libre.

10. Procédé selon l'une quelconque des revendications précédentes, caractérisé en ce que le catalyseur de lipase est présent sous forme d'une enzyme lipase immobilisée sur une matière de support.